Europäisches Patentamt

European Patent Office

Office européen des·brevets

(11) Publication number: **0 172 822**
**B1**

(12) **EUROPEAN PATENT SPECIFICATION**

(45) Date of publication of patent specification: **31.01.90**

(51) Int. Cl.⁵: **C 12 Q 1/00, G 01 N 33/48**

(21) Application number: **84901829.6**

(22) Date of filing: **18.04.84**

(86) International application number:
**PCT/SE84/00148**

(87) International publication number:
**WO 84/04109 25.10.84 Gazette 84/25**

(54) METHOD FOR ANALYSIS OF THE ACTIVITY OF RECEPTORS OF CERTAIN CELLS.

(30) Priority: **19.04.83 SE 8302190**

(43) Date of publication of application:
**05.03.86 Bulletin 86/10**

(45) Publication of the grant of the patent:
**31.01.90 Bulletin 90/05**

(84) Designated Contracting States:
**AT BE CH DE FR GB LI LU NL SE**

(56) References cited:
**EP-A-0 005 032**      **DE-A-2 829 316**
**WO-A-82/03227**      **DE-A-3 038 255**
**WO-A-83/02953**      **DE-A-3 147 763**
**DE-A-2 431 918**      **SE-A-7 909 135**
**DE-A-2 628 468**

(73) Proprietor: **BIO-INSTRUCTA LABKONSULT**
**Söderdalsgatan 32**
**S-502 54 Boras (SE)**

(72) Inventor: **NILSSON, Tomas**
**Söderdalsgatan 32**
**S-502 54 Boras (SE)**

(74) Representative: **Hagelberg, Anders Torvald**
**Sverker et al**
**Albihn West AB Stora Nygatan 15**
**S-411 08 Göteborg (SE)**

## Description

The present invention relates to a method for analysis of receptor activity in a test sample including test cells such as blood cells or a tissue sample, in which a test reaction occurs and produces reaction products at an individually varying optimal ratio between the concentration of the receptors and the concentration of an agent with which the receptors interact.

Within the field of cell biology, knowledge has now been acquired concerning a multitude of reactions which are triggered by receptors on cell surfaces. A number of these receptors react according to an all-or-nothing principle, that is, when optimal conditions exist between the receptor and the agent which acts upon it, a "total reaction" occurs which cannot be immediately repeated again by the same cell. It is often the case that a certain concentration of agent in relation to the concentration of receptors must be attained in order for the reaction to take place. When this threshold concentration is exceeded, the reaction takes place rapidly and completely. In addition to a minimum threshold, other receptors also have a maximum threshold for the amount of agent which can trigger the reaction. This is the case for, e.g., the reactions of mast cells and basophilic leucocytes in allergic individuals, when too much allergen is exposed to the cells.

Another fact in this context is that there is large individual variation in respect of the reaction ability of the receptor and of which concentration ratio is optimal, and also in respect of how the triggered reaction subsequently manifests itself in the form of other reactions in the tissue.

When testing for allergy, in most places, a practical working routine, approximately as follows, is used:

The patient has acquired an idea of his symptoms, and this constitutes the basis of the case history, which is noted by the physician. On the basis of his own experience, the patient is often able to specify the allergen (e.g. grass or cat) and, with special knowledge founded on knowledge of probable allergens in various environments, seasons, etc., the physician can obtain a fairly good idea of which allergens are of interest.

In order to objectify this, tests are available. These tests are of two types: Tests on the patient himself (in vivo tests), and tests of tissue samples, usually blood samples, from the patient (in vitro tests). These two types of tests are, briefly, as follows:

In vivo, the reaction in question may be achieved by imitating the process which causes the symptom. Thus, provocation is caused by having the patient inhale the allergen, whereby hay-fever and asthma may be caused. Drops containing the allergen may be placed into the eyes, causing runny eyes, etc.

The most common in vivo test involves, however, skin tests, of which the most common is the so-called skin puncture test, in which a drop of the allergen in solution or in powder form is applied to the skin, whereupon a slender lancet is stuck through the allergen and underlying skin. After 20-30 minutes the result is obtained as an itchy red patch, the diameter of which is a rough measure of the degree of allergy. In another drop there is usually also added a reference substance (histamine) allowing better judgement of the reaction.

The test is usually performed on the arm in order to allow screening off of the reaction from the rest of the body in case a violent reaction results. It is then easier to treat a possible, potentially fatal, generalization of the reaction (anaphylactic shock).

In this way the so-called Type-1 reaction (hay-fever, certain forms of asthma and rashes, red eyes and hypersensitivity towards certain drugs, etc.) may be objectified.

In these tests, the allergen is allowed to diffuse into those cells which have receptors on their surfaces which are directed towards the allergen. These cells are the so-called mast cells in the tissues. Their counterparts in the blood system are the so-called basophilic leucocytes, which are few in number in comparison to other blood cells (around 0.5% of all leucocytes or white blood cells).

When the mast cell or the blood basophile meets with an allergen under certain conditions, it empties its content of small granules, granula, from which histamine is then liberated, and causes the symptoms as it reacts with histamine receptors of other cells.

A number of tests may also be carried out in vitro in order to objectify an allergy. The most common method in Sweden is the PRIST®-test (Paper Radio Immune Sorbent Test, Pharmacia), in which the proportion of IgE-molecules is measured. It is usually higher in allergic persons than in healthy persons. IgE molecules constitute a part of the receptor on the mast cells and basophilic leucocytes for the allergen. IgE is formed by lymphocytes, particularly when they are stimulated by an allergen. Lymphocytes release allergens into the body fluids and a small number of these allergens adhere to the mast cells and basophilic leucocytes (also to other cells in certain cases). Less than 0.1% of IgE is bound to the reacting basophilic leucocytes, but there is probably a very large variation in this among different individuals.

Thus, positive PRIST does not mean that an allergic reaction has been measured, but use is made of the fact that many, but not all, allergic persons have a higher proportion of IgE in the blood than do healthy persons. It is also possible to measure the proportion of specific IgE for a large number of allergens. The test is then known as RAST® (Pharmacia). There is often a good correlation between the results of RAST and the results of a skin puncture test, at least if the same allergen is used in the reagent of both tests.

These two tests are used to a varying extent to complement the case history and the skin puncture testing.

Relatively little blood is consumed for tests which utilize radiological technology, which is a common technique for analysis at larger hospitals in Sweden. The cost for such tests, however, is so high that, most often, only in vivo-tests are carried out.

There are also other methods for in vitro testing for allergy. For research purposes the release by mast cells and basophilic leucocytes of histamine or other substances upon contact with allergens is used, but this methodology is complicated and expensive, with results which are often difficult to judge, since the proportion of histamine in the blood of healthy people can not yet be reliably determined and great difficulties are encountered when directly measuring possible changes in a blood sample. In practically all known methods it has been found necessary to first enrich and wash the cells before reaction, which is then correlated with how much total histamine there is in the cells, which requires large volumes of blood per allergen.

Especially in France, techniques have also been used wherein the basophilic leucocytes have been investigated in connection with allergy. Briefly, the methodology has been approximately as follows, which also applies if measurements of the release of histamine have been made: A blood sample is divided into portions, possibly after a simple enrichment step. To each portion, a specific proportion of allergens is added. Usually six portions containing a few ml basophilic suspension are used, to which is added a series of concentrations of allergen (to the extent that the reactive part of the allergen extract can be measured), from about lng/ml in steps of a power of 10 up to 1 mg/ml. This varies to some extent with the allergen preparation, but it has been found necessary to cover a spectrum of 1 million concentration units due to individual variations. After a waiting period of about half an hour the basophilic leucocytes are then counted in dried and colored blood smears in a microscope.

Due to the large amount of work involved, this has not been widely used as a method for allergy tests, despite the fact that the actual reaction of the effector cell in allergy response is measured for a sample delivered to a hospital laboratory instead of on the patient, as is the case for a skin puncture test.

Very recently, a couple of publications have described the use of high-capacity cell counters in the evaluation of the reaction, but still the necessary blood volume is large if many allergens are tested, since several portions are used for each allergen tested.

There are several advantages to be had by using basophilic leucocytes to diagnose allergy:

In principle the same initial effector mechanism is studied as the one which causes the symptoms in the patient.

Blood samples can be sent to the laboratory instead of the patient having to take time off from work in order to occupy a hospital waiting room as is the case with skin puncture testing.

The risk of anaphylactic shock or of other reactions during the testing disappears. Certain drugs and other potentially dangerous allergens may be tested without injury to the patient.

Only the actual allergic reaction is studied, other unspecified types of reactions being eliminated, and the diagnosis becomes more reliable.

The object of the invention and its most important characteristics

The present invention provides a method which permits clinical utilization of reactions of the type mentioned above, e.g., for allergy diagnostics, where the required amount of the sample, i.e., the blood volume, and the amount of work for the execution of the analysis, are minimal, and the method is suitable for automatization.

This has been achieved by exposing the cells in one single sample to a concentration of agent which is increased either continuously or stepwise by continuous or discrete addition of the agent so that said optimal ratio is present for a period of time which is sufficiently long for the test reaction to take place, whereupon the sample is analysed with respect to the reaction products. If the concentration of agent then exceeds the optimal it will not matter, since cells will already have reacted.

Theoretical background:

The mechanism behind the reaction between basophilic leucocytes and allergens may be described in the following manner:

On the cell surface there are receptors for IgE-molecules, which are produced by lymphocytes and are released into the body fluids, which, in all human beings, contain a small amount of IgE. In allergic persons the proportions are often much higher (cf. PRIST® and RAST® tests).

There is an equilibrium between the number of free IgE-molecules and the number bound to the mast cell and to the cell. Each cell has a large number of IgE-receptors (40-500 000 per cell).

IgE reacts with allergen in such a way that several IgE are bound together. Both free and cell-bound IgE bind allergen, but only the bound ones produce further reaction.

The proportion of receptors per cell, the relative number of IgE-molecules bound, the ratio between free and bound, the affinity of IgE towards the allergen, and the ability for further reaction vary from individual to individual. This means that the amount of allergen which must be added to a sample of cells, or through the skin as in skin puncture testing, varies considerably with the individual. In addition, the requirement for not too much yet not too little allergen must be satisfied.

The reaction between allergen and IgE must be effected in the following way in order for continued reaction to be triggered: Each IgE bound to the cell surface can react with the allergen, but only a small portion of all IgE is needed in order for the reaction to take place, perhaps on the order of tens of IgE-molecules. The triggering factor is the change which occurs around the IgE-molecule in the cell membrane which is caused when an allergen simultaneously binds together two or possibly more IgE-molecules.

If there are too few allergens in relation to the IgE then two IgE will not be able to simultaneously bind the same allergen, and nothing further occurs. If there are too many allergen molecules which can bind IgE, each IgE will bind allergen, but the probability of two simultaneously binding the same allergen is reduced. When the ratio between allergen and IgE is optimal, i.e. two or several IgE are bound together by allergen, then the following occurs:

Calcium ions can leak in through a "hole" in the cell membrane, which is held open for a short time, probably a period of seconds. If enough calcium enters the cell, mechanisms are triggered which, within a couple of minutes, will eject the granula of the cell from it. Histamine and other substances are released from these and the allergic reaction begins.

The reaction may be registered in various ways, of which one way consists of counting the basophilic leucocytes, excluding the degranulated cells. Nowadays there are automatic devices for counting the basophilic leucocytes, e.g. TECHNICON® H6000. For research purposes methods are also used in which released substances are metered as a measure of the reaction. These methods are, however, presently laborious and expensive.

The reaction described above occurs very rapidly and it is crucial that binding together of at least two IgE per allergen take place. This binding is a very fast reaction whereas the release of histamine subsequently takes a few minutes. Once the cell has reacted, it is emptied of granula and cannot react further or (as far as is known, at least in human beings) absorb granula again. It does not matter therefore if the concentration of allergen is too high as long as the reaction has already taken place.

This means that the cells of one single blood sample may be exposed to an increasing concentration of allergen, up to a predetermined final concentration, provided that the time, during which the optimal concentration of allergen for the reaction is present, is sufficiently long, but not too long, since the reaction may then possibly be inhibited. Thus, the optimal interval should be passed through over the course of a few minutes, after which the allergen concentration may rise to very high levels without affecting degranulation which has already taken place. This resembles the natural process in the body when the allergen diffuses to the receptor.

Examples of practial solutions of the inventive method:

1. Stepwise addition of allergen to a blood sample, thus increasing the concentration stepwise, either manually, or automatically using pippettes, in which case there is a time interval between the addition steps.

From the patient is taken 10 ml of blood in a Vacutainer tube having EDTA added, which is mixed by rocking the tube. To this volume of blood is added 20-25 µl Heparin solution (Lövens injection liquid Heparin 5000 IE/ml, diluted to 500IE/ml with isotonic sodium chloride).

The tube is rocked until mixing has taken place.

In certain cases, up to week-old EDTA-blood may be used if the sample has been stored in a refrigerator, possibly after addition of certain nutritive substrates.

The blood is divided in portions of 300 µl each in suitable tubes, e.g., polyethylene tubes. 10 ml is enough for about 30 portions and thus, in principle, for 30 allergy tests. 20µl of 0.06 M $CaCl_2$, and possibly 0.03 M $MgCl_2$-solution, is added to one of the tubes. This is not added to a second tube (a control tube).

Allergen, e.g., from DOME® (skin puncture test solution, e.g. five-grass mix of 4000 PNU/ml in 50% glycerine) is diluted in a number of tubes as follows:

Diluent: Albuminė solution 16 g/l (Kabi, albumine 200 mg/ml diluted with a sterile isotonic sodium chloride solution).

Solution 6: Extract diluted 1+1 with diluent.
Solution 5: The above diluted 1+9 with diluent.
Solution 4: The above (5) diluted 1+9 with diluent.
Solution 3: The above (4) diluted 1+9 with diluent.
Solution 2: The above (3) diluted 1+9 with diluent.
Solution 1: The above (2) diluted 1+9 with diluent.

Further dilution may possibly be necessary.

The blood samples are placed in a water bath at 37°C.

20 µl of these solutions is subsequently added to the two test tubes in the water bath with an interval of a few minutes between each addition, starting with the most diluted solution. When the most concentrated solution has also reacted for a period of a few minutes, the reaction is interrupted using an excess of EDTA solution, e.g. 50 µl 10% $K_3$EDTA in water, whereby calcium ions are bound.

The tubes are then placed in a TECHNICON® H6000 apparatus or in some other detector for measuring the reaction.

A test is considered positive if the basophilic leucocytes of the patient have been reduced in number, often disappearing completely, as measured by the detector. (In the control tube without calcium ions the number will not have been reduced).

2. Automatic allergen addition in a flow system:

"Continuous-flow" equipment is arranged so that a blood sample is sucked into a hose at a certain rate (e.g. 500 µl/minute) and then passes "stations" with addition of Ca-Mg solution and allergen in progressively increasing concentration at certain intervals of time. When the sample appears at the other end of the system, EDTA is also added and the sample may be collected in a tube which is then placed in the detector.

It is possible in this case to make a direct connection to an automatic detector system, e.g., the basophilic channel of an H6000-apparatus.

3. Continuous addition of allergen to the blood sample by diffusion or by chemical reaction with it:

On one end of a suitable carrier such as a stick of inert plastic material, a device is placed which continuously releases increasingly higher concentrations of allergen, or of other agents for other types of receptor analysis which are based on this principle.

The release process in the sample must take place in such a way that the optimal interval is passed through during a sufficient, but not too long, length of time. A stick suitable for the purpose may be made of, e.g., GELBOND® or drawing film, one side of which is hydrophilic. Agarose can be bound to this surface. The agarose can then be used to bind allergen solution, whereby the allergen diffuses into the agarose. If additional, possibly more concentrated agarose or gelatine is placed on top of the previous agarose, after it has dried, a concentration gradient can; by means of diffusion, be set up within a very thin layer of agarose, when everything has dried after the second diffusion. On top of this, gelatine having a lower concentration of allergen may for example also be allowed to dry. If this stick is placed in a tube containing blood, or in a suitable liquid with a suspension of cells from allergic persons, and if a suitable concentration of agarose has been used, a degranulation reaction will occur. Calcium is added to the sample (or deposited in advance on the stick in a rapidly soluble form), whereupon the stick is positioned in the tube for a given length of time. Thereafter, it is removed and, after final addition of EDTA, the sample is ready for the detector. This method resembles the diffusion of allergen to the mast cells in a skin puncture test.

Example of production of sticks for testing for grass, broad-leaf tree, daisy, mite, dust, cat, and horse on a number of allergic persons:

DOME skin puncture test allergen as above is used, taken directly from the package.

A warm 0.25% agarose solution (SEAKEM) in water is poured onto a hydrophobic surface of a layer of about 3 mm, which is positioned horizontally. After solidification, cylinders are punched out having the same diameter as the width of the GELBOND® stick and are placed immediately adjacent to one end, on the hydrophillic side. On this gel cylinder is placed 3 µl of skin puncture test solution and this is allowed to diffuse while a heating fan is used to carefully dry the gel, or alternatively, it is positioned in such a way that the gel hangs over the edge of a table and is allowed to dry. When the cylinders have dried up to a spot, a similar cylinder is placed on top of the spot and the evaporation step, with simultaneous back diffusion of the allergen, is repeated. The stick is then ready for use.

Take e.g. 400 µl of blood with additives, or a cell suspension. Add calcium solution according to the previously described manual method, 20 µl 0.06M CaCl$_2$ and possibly 0.03 M MgCl$_2$. Place the tube in a water bath at 37°C. Place a stick in the tube and shake it occasionally, or stir with the stick so that the blood is brought into contact with the whole agarose surface, at least for short periods of time. Remove the stick after about 30 minutes and add EDTA in order to stop the reaction by binding calcium. The tube is then placed in the detector. A positive reaction is defined as above.

The stick indicated in the example has been tested by letting radioactively marked protein, having a molecular weight of 22 000 (most allergens are of this size), diffuse into the blood as described. After 30 minutes, 90% of the radioactivity was located outside the stick; after 5 minutes approximately a third. By modification of the binding substance, possibly by using protein binding, of the allergen, etc, this can be varied.

Continuous addition by diffusion or elution may also be accomplished using a substance which is itself dissolved in the blood. The simplest method employs a "tablet" made of, e.g., gelatine, which dissolves completely and disappears as an indication that the reaction is finished.

The allergen may also be bound to an insoluble substance, e.g., to a tablet of the "slow release" type, common in connection with pharmaceuticals.

The allergen may also be dissolved from the tube itself or from a stopper which fits into the tube, which, after being stopped up, is turned upside down to allow the reaction to occur and, after "unstopping", is placed in the detector.

It is also possible to envision a stopper such as a cork which fits the tube and which is designed as a container. A paper strip or a capillary containing an absorbant material, such as a thread which has been soaked in allergen solution with suitable additives and dried, is attached to the cork. The tube with blood is corked using this device. The capillary should reach down to the blood. Eluation solution is then poured into the cork and is absorbed by the thread, whereby the allergen successively is released at the other end of the capillary and into the blood.

The allergen may also be added to the sample chemically bound to, e.g., a protein, from which it is successively released to the sample by way of chemical reaction.

The method according to the invention is naturally not limited to the embodiments described above or to the reaction of basophilic leucocytes with allergen. Examples of other kinds of diseases which can be diagnosed by means of the method described are diseases in connective tissues and rheumatoid arthritis, whereby one makes use of the reaction of basophilic leucocytes with an extract of nucleus cells or DNA molecules in pure form. Other fields of application may be the study of the germicidal ability of neutrophilic leucocytes in patients with defective immune defense (e.g. cronic granulating disease, myeloperoxide deficiency, and others) and the reaction of trombocytes on a stimulus of the collagen type or on the products of the protease cascade systems.

## Claims

1. Method for analysis of receptor activity in a test sample including test cells such as blood cells or a tissue sample, in which a test reaction occurs and produces reaction products at an individually varying optimal ratio between the concentration of the receptors and the concentration of an agent with which the receptors interact, characterised in that the concentration of the agent to which the test cells are exposed is increased either continuously or stepwise by continuous or discrete addition of the agent so that said optimal ratio is present for a period of time which is sufficiently long for the test reaction to take place, whereupon the sample is analysed with respect to the reaction products.

2. Method as claimed in claim 1 in which the receptors consist of IgE molecules bound to the cell surface of basophilic leucocytes and the agent consists of molecules or particles of allergen and in which a positive test reaction is such that, at the optimal ratio between the concentration of the IgE molecules and the concentration of the allergen, basophilic degranulation of the test cells, with accompanying release of histamine, occurs, being initiated by ions such as $Ca^{2}+$, characterised in that allergen is added continuously or stepwise to the test sample so that the concentration of the allergen is gradually increased to a predetermined level and in that said increase in the concentration of allergen is carried out over a period of time which is long enough to allow the positive test reaction to take place at the optimal allergen concentration, and in that, after completed addition of allergen, the test sample is analysed by classifying the basophilic leucocytes with respect to their granulation or, alternatively, by measuring histamine or other substances which are released upon degranulation or the effects thereof on other measurable processes.

3. Method as claimed in claims 1 or 2, characterised in that the agent is added to the test sample bound to a carrier from which it is gradually released by means of diffusion, elution or by chemical reaction.

4. Method as claimed in claims 1 or 2, characterised in that the agent is added to the test sample gradually in increasing concentration at predetermined time intervals.

## Patentansprüche

1. Verfahren zur Analyse der Rezeptoraktivität in einer Probe, enthaltend Testzellen wie Blutzellen oder eine Gewebeprobe, bei welchem eine Testreaktion stattfindet und Reaktionsprodukte bei einem im einzelnen variierenden optimalen Verhältnis zwischen der Konzentration der Rezeptoren und der Konzentration eines Mittels, mit welchem die Rezeptoren in Wechselwirkung treten, entstehen, dadurch gekennzeichnet, daß die Konzentration des Mittels, welchem die Testzellen ausgesetzt werden, entweder stetig oder schrittweise durch stetige oder diskrete Zugabe des Mittels erhöht wird, so daß das optimale Verhältnis während eines Zeitraums vorliegt, der ausreichend lang ist, um die Testreaktion ablaufen zu lassen, worauf die Probe bezüglich der Reaktionsprodukte analysiert wird.

2. Verfahren nach Anspruch 1, bei welchem die Rezeptoren aus an die Zelloberfläche von basophilen Leukozyten gebundenen IgE-Molekülen bestehen und das Mittel aus Allergenmolekülen oder Allergenteilchen besteht und bei welchem eine positive Testreaktion derart gestaltet ist, daß beim optimalen Verhältnis zwischen der Konzentration an IgE-Molekülen und der Konzentration des Allergens eine basophile Degranulierung der Testzellen, verbunden mit der Freisetzung von Histamin, stattfindet, welche durch Ionen wie $Ca^{2}+$ initiiert wird, dadurch gekennzeichnet, daß das Allergen stetig oder schrittweise zur Probe zugegeben wird, so daß die Konzentration des Allergens graduell auf ein vorbestimmtes Niveau erhöht wird und daß die Zunahme der Allergenkonzentration über einen Zeitraum vorgenommen wird, welcher ausreichend lang ist, um die positive Testreaktion bei der optimalen Allergenkonzentration ablaufen zu lassen und daß nach vollständiger Zugabe des Allergens die Probe durch Klassifikation der basophilen Leukozyten bezüglich ihrer Granulierung oder alternativ hierzu durch Messung von Histamin oder anderer Substanzen, die nach der Degranulierung freigesetzt werden oder durch Messung ihrer Wirkungen auf andere meßbare Vorgänge analysiert wird.

3. Verfahren nach Anspruch 1 oder 2, dadurch gekennzeichnet, daß das an einem Träger gebundene Mittel zur Probe zugegeben wird, von welchem es graduell durch Diffusion, Elution oder durch eine chemische Reaktion freigesetzt wird.

4. Verfahren nach Anspruch 1 oder 2, dadurch gekennzeichnet, daß das Mittel graduell in zunehmender Konzentration zu vorbestimmten Zeitintervallen zur Probe zugegeben wird.

## Revendications

1. Procédé pour l'analyse de l'activité des récepteurs dans un échantillon d'essai comprenant des cellules d'essai, telles que des globules du sang ou un échantillon de tissu, dans lequel une réaction d'essai a lieu et engendre des produits de réaction à un rapport optimal individuellement variable entre la concentration des récepteurs et la concentration d'un agent avec lequel les récepteurs réagissent, caractérisé en ce que la concentration de l'agent auquel les cellules d'essai sont exposées est augmentée, de façon continue ou par paliers, par addition continue ou discontinue de l'agent de sorte que ledit rapport optimal est présent pendant une durée qui est suffisamment longue pour que la réaction d'essai ait lieu, après quoi on analyse l'échantillon en ce quiconcerne les produits de la réaction.

2. Procédé suivant la revendication 1, dans lequel les récepteurs sont des molécules d'IgE liées à la surface de cellule de leucocytes basophiles et l'agent consiste en molécules ou particules d'allergène, et dans lequel une réaction d'essai positive est telle que, au rapport optimal entre la concentration des molécules d'IgE et la concentration de l'allergène, il se produit une dégranulation basophile des cellules d'essai, accompagnée d'une libération d'histamine, cette dégranulation étant déclenchée par des ions tels que $Ca^{2+}$, caractérisé en ce qu'on ajoute l'allergène de façon continue ou par paliers à l'échantillon d'essai de sorte que la concentration de l'allergène augmente progressivement jusqu'à un niveau prédéterminé, et en ce qu'on effectue ladite augmentation de la concentration de l'allergène en un laps de temps qui est assez long pour permettre à la réaction d'essai positive de se produire à la concentration optimale d'allergène, et en ce que, après la fin de l'addition d'allergène, on analyse l'échantillon d'essai par classement des leucocytes basophiles en fonction de leur granulation ou, en variante, par mesure de l'histamine ou d'autres substances qui sont libérées lors de la dégranulation, ou de leurs effets sur d'autres processus mesurables.

3. Procédé suivant la revendication 1 ou 2, caractérisé en ce que l'agent est ajouté à l'échantillon d'essai lié à un support à partir duquel il est progressivement libéré par diffusion, élution ou réaction chimique.

4. Procédé suivant la revendication 1 ou 2, caractérisé en ce que l'agent est ajouté à l'échantillon d'essai progressivement, à concentration croissante, à intervalles de temps prédéterminés.